# EUROPEAN PATENT APPLICATION

(11) **EP 4 846 945 A1**
(43) Date of publication of application: **02.12.2026**
(21) Application number: 25178849.3
(22) Date of filing: 26.05.2025
(51) Int. Cl.: A61B 8/08, A61B 8/00

(54) **ULTRASOUND IMAGING SYSTEM FOR PROVIDING HARMONIC B-MODE IMAGING AND FUNDAMENTAL MODE ATTENUATION IMAGING**

(71) Applicant: Koninklijke Philips N.V., 5656 AE Eindhoven (NL)
(72) Inventor: XIE, Hua, Eindhoven (NL); NGUYEN, Man Minh, Eindhoven (NL)
(74) Representative: Philips Intellectual Property & Standards

(57) **Abstract**

An ultrasound system (200) for tissue attenuation imaging is disclosed. When executed by a processor (220), computer executable instructions cause the processor to receive radio frequency (RF) data including a fundamental mode and a harmonic mode from an ultrasound probe (245). The harmonic mode includes a fundamental component and a harmonic component. The system includes a brightness (B) mode signal path (306), and a B-mode image is generated with the harmonic mode component. An attenuation imaging map (532, 622) is generated from the fundamental mode component. The B-mode image (520, 620) and the attenuation imaging map (532, 622) are superimposed, and are provided on a display.

## Description

### FIELD OF THE INVENTION

The present teachings relate to an ultrasound system and to a method adapted to provide improved B-mode imaging, and attenuation imaging properly calibrated to provide reliable acoustic attenuation coefficients for the tissue being imaged.

### BACKGROUND OF THE INVENTION

Pulse-echo ultrasound imaging systems transmit beams of acoustic energy over an image field. As each transmitted beam encounters acoustic reflectors and tissue boundaries, some of the transmitted energy is reflected back to the transmitting transducer and received as an echo. In this way, a sequence of echoes is received from progressively deeper tissues as the ultrasound beam energy travels deeper into the body. The amplitudes of the echoes are detected and displayed in correspondence with their times of reception, which correspond to the depths from which they were received. The display thereby reveals characteristics of the structure of the tissue within the body. But the beam energy is continually attenuated as it travels through the tissue and encounters acoustic scatterers along the paths of the beams. This attenuation will result in the reception of generally stronger echoes from tissue in the near field, and generally weaker echoes from tissue at greater depths.

The acoustic attenuation coefficient (α) is a fundamental tissue property. The acoustic attenuation coefficient represents the intrinsic ability of a medium to reduce the intensity of sound waves as they propagate through it. The acoustic attenuation coefficient is measured in dB/cm/MHz, quantifies how much the intensity of an ultrasound wave decreases as the wave travels through a medium, primarily due to absorption and scattering. Attenuation is primarily caused by absorption (conversion of sound energy to heat) and scattering (sound waves changing direction) within the tissue.

Different tissues have varying attenuation coefficients. For example, bone has a much higher attenuation coefficient than soft tissues like muscle or liver. Moreover, higher frequency ultrasound waves are generally attenuated more than lower frequency waves.

In medical applications, the acoustic attenuation coefficient of tissue usefully correlates with the pathology of the tissue. For example, the liver acoustic attenuation coefficient of liver tissue increases from healthy liver to fatty liver. As such, a fatty liver is more attenuative than normal liver tissue. As more fat droplets accumulate inside the liver cells, the acoustic attenuation coefficient of the liver tissue will increase. Numerous clinical studies have demonstrated a strong correlation between acoustic attenuation measurement and liver biopsy (gold standard for liver steatosis assessment) or MRI-PDFF (proton density fat fraction, a new reference method). Ultrasound attenuation Imaging provides real-time 2D mapping and quantification of local tissue acoustic attenuation coefficient. It holds promise as a quantitative metric for non-invasive and cost-effective liver steatosis staging and assessment.

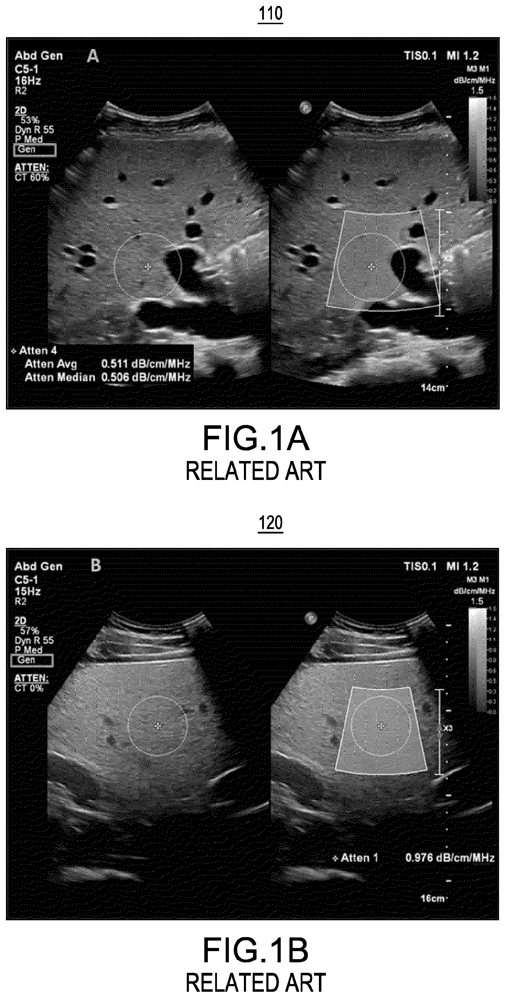

Known ultrasound (US) imaging systems thus may be used to determine the attenuation coefficient of tissue for certain diagnoses/pathologies. These known US systems measure the acoustic attenuation coefficient using the fundamental imaging mode. However, the image quality realized using fundamental mode imaging characteristically provides comparatively poor clarity, and as a result, harmonic mode imaging is preferred for regular B-mode tissue imaging. Figs. 1A and 1B provide illustrative attenuation imaging featuring a pair of normal liver and fatty liver, respectively. Normal liver has acoustic attenuation coefficient value of 0.511 dB/cm/MHz, whereas the fatty liver has higher attenuation measurement of 0.976 dB/cm/MHz. The imaging mode for the B-mode is fundamental mode as indicated by the annotation "Gen" in the images.

As noted above, the US signal power attenuates with, among other factors, the increasing depth in the tissue, so a plot of signal power along depth provides a downward slope, with the attenuation coefficient being calculated by the slope of this line. To properly determine the attenuation coefficient, the determined slope is measured against a reference slope, with the difference between the slope of the tissue and the reference slope providing the more accurate attenuation of the US signal. Currently, fundamental imaging for attenuation imaging includes calibration using a phantom with known acoustic attenuation coefficient. Calibration in fundamental imaging mode is understood as a useful step in estimating tissue parameters as it helps remove system dependence (such as beam diffraction pattern). Moreover, tissue and reference phantom may experience different non-linear effects during wave propagation. As such, prior knowledge in the literature may be used to establish that the difference in beam diffraction pattern between tissue and reference phantom is smaller in fundamental imaging mode than in harmonic imaging mode. As a result, fundamental mode attenuation imaging compromises image quality (IQ) of the underlying B-mode image.

Accordingly, current attenuation imaging is based on fundamental mode imaging at the expense of B-mode image quality. By contrast, harmonic imaging methods are known to provide an improved image quality over fundamental imaging methods. However, generating attenuation images and determining the attenuation coefficient using harmonic mode imaging has not been adapted for various reasons. For example, when using harmonic mode imaging, it has been found that the phantom image and the tissue image may have different harmonic imaging characteristics, which may reduce or prevent system dependence by using a phantom as a reference in harmonic imaging. As such, while image quality may be improved using an US device based on harmonic mode imaging, the inability to properly calibrate the US system using harmonic phantom images eliminates the ability to provide B-mode imaging and attenuation imaging in a side-by-side manner as in Figs. 1A and 1B using harmonic mode imaging.

What is needed, therefore, is a US system that overcomes at least the drawbacks of known fundamental and harmonic mode imaging systems described above.

### SUMMARY OF THE INVENTION

The invention is defined by the independent claims. Advantageous embodiments are provided in the dependent claims.

According to a representative embodiment, an ultrasound system for tissue attenuation imaging is disclosed. The ultrasound system comprises an ultrasound probe. The ultrasound system further comprises: a processor; and a tangible, non-transitory computer readable medium that stores instructions. When executed by the processor, the instructions cause the processor to: receive radio frequency (RF) data from the ultrasound probe, the RF data comprising a fundamental mode and a harmonic mode; separate the RF data comprising the fundamental mode and the harmonic mode to provide a fundamental component and a harmonic component, respectively; create a brightness (B) mode signal path, and generate a B-mode image based on the harmonic mode component; generate an attenuation imaging map based on the fundamental mode component; superimpose the B-mode image and the attenuation imaging map; and display the superimposed B-mode image and the attenuation imaging map of a tissue attenuation image.

According to another representative embodiment, a tangible, non-transitory computer-readable medium that stores instructions is disclosed. When executed by a processor, the instructions cause the processor to: receive radio frequency (RF) data from the ultrasound probe, the RF data comprising fundamental and harmonic components; separate the RF data to provide a fundamental frequency component and a harmonic frequency component, respectively; create a brightness (B) mode signal path, and generate a B-mode image based on the harmonic mode component; create an attenuation signal path, and generate an attenuation imaging map based on the fundamental frequency component; superimpose the B-mode image and the attenuation imaging map; and display the superimposed B-mode image and the attenuation imaging map.

By superimposing the B-mode image and the attenuation imaging map to display a tissue attenuation image, the visualization of tissue characteristics is greatly enhanced.

In some embodiments, before the superimposing, the instructions further cause the processor to calibrate the attenuation imaging map using a set of reference data acquired from a phantom having a known acoustic attenuation coefficient. By calibrating the attenuation imaging map using reference data from a phantom with a known acoustic attenuation coefficient prior to superimposing the B-mode image and the attenuation imaging map may advantageously ensure accurate and reliable imaging results.

Preferably, the calibration comprises acquiring calibration data in a harmonic imaging mode. Acquiring calibration data in a harmonic imaging mode may advantageously improve the accuracy of the calibration process, leading to more precise imaging results.

More preferably, the instructions further cause the processor to separate the fundamental component from a broadband signal and average the fundamental component over multiple calibration data. It has been realized that separating the fundamental component from a broadband signal and averaging it over multiple calibration data points may enhance the consistency and reliability of the calibration, which results in more accurate imaging.

In some embodiments, intensities of the fundamental component for different frequency bands are used to eliminate system dependencies. That is, in such embodiments, the instructions may further cause the processor to extract the intensity of the fundamental component for different frequency bands. In this way, system dependencies can be advantageously eliminated, ensuring that the imaging results are not affected by variations in the system's performance, leading to more consistent and reliable imaging.

In some embodiments, the phantom may be a uniform phantom. The use of a uniform phantom may ensure consistent and reliable calibration of the attenuation imaging map, as the phantom has uniform properties throughout. In particular, as the uniform phantom has a known acoustic attenuation coefficient, a standard reference for calibration can be provided, which further enhances the accuracy of the attenuation imaging map.

In some embodiments, the instructions further cause the processor to generate the harmonic component by providing a pair of ultrasound pulses that are inverted 180° out of phase, and sum the ultrasound pulses to substantially cancel the fundamental component. In this way, the fundamental component can be advantageously cancelled, which allows for clearer harmonic imaging by reducing noise from the fundamental component.

Preferably, the instructions may further cause the processor to subtract the pair of ultrasound pulses to obtain the fundamental component and cancel the harmonic component. This may be particularly advantageous to ensure that the fundamental component is isolated, which improves the accuracy of attenuation imaging.

Also preferably, the pair of ultrasound pulses may be provided in sequence to one another. That is, in such embodiments, the instructions may further cause the processor to provide the pair of ultrasound pulses in sequence to one another. This may advantageously ensure that the pulses are correctly timed, enhancing the effectiveness of the harmonic and fundamental component separation.

According to another representative embodiment, a method for tissue attenuation imaging is provided. The method may preferably be computer implemented. The method comprises: receiving radio frequency (RF) data from an ultrasound probe of an ultrasound system, the RF data comprising a fundamental mode and a harmonic mode; separating the RF data comprising the fundamental mode and the harmonic mode to provide a fundamental component and a harmonic component, respectively; creating a brightness (B)-mode signal path, and generating a B-mode image based on the harmonic component; creating an attenuation signal path, and generating an attenuation imaging map based on the fundamental component; superimposing the B-mode image and the attenuation imaging map; and displaying the superimposed B-mode image and the attenuation imaging map of a tissue attenuation image.

Embodiments of the present invention described above in the context of the ultrasound system or the tangible, non-transitory computer-readable medium can be equally implemented as a corresponding method and, in particular, a computer-implemented method.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Figs. 1A-1B show side-by-side images of a normal liver and a fatty liver, respectively, from a known US imaging system.
Fig. 2 is a simplified block diagram of a US imaging system in accordance with a representative embodiment.
Fig. 3 is a flow-chart showing the function of the US imaging system of Fig. 2 in accordance with a representative embodiment.
Figs. 4A-4C show power spectra of a reference phantom generated for system calibration in accordance with a representative embodiment.
Fig. 5A show a US B-mode image generated using fundamental mode imaging.
Fig. 5B shows a B-mode image generated using harmonic mode imaging in accordance with a representative embodiment.
Fig. 5C shows an attenuation map derived from the fundamental signals and superimposed on the B-mode harmonic image of Fig. 5B in accordance with a representative embodiment.
Figs. 6A and 6B are US images showing equivalent attenuation coefficient estimation performance between the known feature using a known US imaging system, and a US imaging system in accordance with a representative embodiment.
Fig. 7 is a flow-chart of a method of performing a US exam in accordance with a representative embodiment.

### DETAILED DESCRIPTION OF EMBODIMENTS

Any of the steps described in relation to examples and/or training described below can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. As an example, a computer system performs logical processing based on digital signals received via an analog-to-digital converter.

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the present teachings relate to generating high-quality B-mode US images using harmonic signals, and accurate acoustic attenuation coefficient estimation using fundamental signals. As described in connection with various representative embodiments, harmonic imaging mode-based US systems are used to improve the attenuation imaging feature with the attenuation imaging being derived from the fundamental signals, maintaining the quantification performance. Harmonic imaging offers superior imaging quality than fundamental imaging in several attributes. Harmonic imaging has better spatial resolution, higher contrast-to-noise ratio, and fewer artifacts such as reverberation and side lobes. Of course, harmonic image also entails certain drawbacks including lower SNR (signal-to-noise ratio) and penetration performance.

Harmonic imaging mode as used in accordance with the present teachings provides access to both fundamental and harmonic RF signals. There are several methods to realize harmonic imaging. One type of imaging in the harmonic mode involves a pulse inversion sequence for standard tissue B-mode imaging. As the name implies, two pulses in a pair with an inverted 180° phase are sent sequentially into the tissue along the same line. At the receive, summation of two pulse echoes will cancel out the fundamental signal but retain the second harmonic signal. Another known approach to B-mode image formation is to combine fundamental imaging and harmonic imaging as the default mode for abdominal imaging. As described more fully below, so-called standard imaging mode for tissue B-mode is referred to as "HGen," which blends fundamental signals and harmonic signals to achieve good tradeoff of spatial resolution and penetration. While these various modes of signal processing are contemplated to provide the harmonic components for the B-mode images, and the fundamental mode for attenuation imaging and acoustic attenuation coefficient determination, the various representative embodiments described below are based on the noted HGen imaging.

As described more fully below, the side-by-side B-mode companion is formed from the harmonic signals, with the benefits of IQ enhancement like improved spatial resolution, enhanced contrast-to-noise ratio, and reduced artifacts. To overcome IQ issues, the harmonic imaging mode-based solutions of the present teachings beneficially improve the tissue attenuation imaging feature. Specifically, attenuation imaging is still derived from the fundamental signals, maintaining their quantification performance. As alluded to above, the drawback of using fundamental imaging mode for attenuation imaging feature is the low image quality of the underlying B-mode. The harmonic imaging mode-based solutions of the present teachings improve the underlying B-mode IQ derived from harmonic components while maintaining the accuracy of attenuation quantification derived from fundamental components.

Accordingly, and as described more fully below, the present teachings comprise three core system components. First, a harmonic imaging mode is implemented to provide access to both fundamental and harmonic RF signals at the receiver. Second, decoupled signal paths are used to create the attenuation image and the B-mode image. Third, system calibration for attenuation coefficient calculation using the fundamental signals is derived from the harmonic imaging mode. As will become clearer as the present description continues, these core system components may be instantiated, at least in part, in computer-executable instructions stored in memory. As described more fully below, the various representative embodiments comprise execution of computer-executable instructions using a processing unit comprising a processor. In this way, the various representative embodiments offer clear improvements to a computer of the US imaging systems described herein.

Among other improvements to the function of an ultrasound system and computer, and other technical fields, the present teachings enable an improved image quality via harmonic imaging and without compromising the attenuation imaging quality by separating the input receive signal in two paths-the first path being the harmonic signal path to create the underlying B-mode image, and the second path being the fundamental signal path to determine the acoustic attenuation coefficient and generate the attenuation image. In this way, the US systems of the various representative embodiments use a single input signal to provide a high-quality B-mode image and an accurate acoustic attenuation coefficient.

Furthermore, the separation of the fundamental and harmonic signals and their processing described in connection with various representative embodiments to provide high-quality B-mode images and the accurate determination of acoustic attenuation coefficients cannot be done by a human mind to duplicate the signal processing to realize these outputs. Clearly, such signal processing and computations could not be done by the human mind with accuracy, or practically be done in the human mind (e.g., within a certain period of time), but even if it could be done in a matter of days if not months, the delivery of any diagnosis would be too late to have meaningful use in the diagnosis and treatment of various medical conditions and diseases that are diagnosed from the high-quality B-mode images generated and accurate attenuation coefficients that are determined in accordance with the US systems of the various representative embodiments of the present teachings.

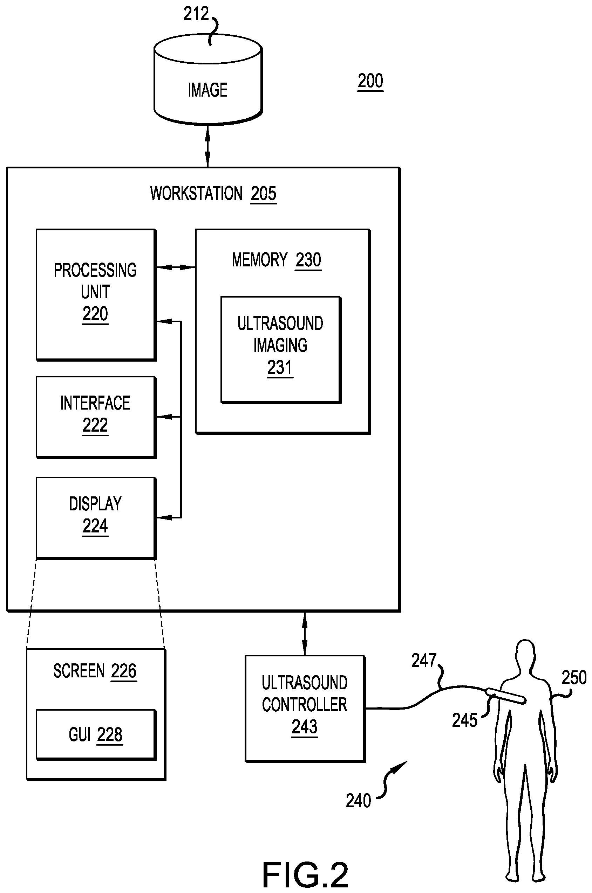

Fig. 2 is a simplified block diagram of an ultrasound (US) system 200 according to a representative embodiment.

Referring to Fig. 2, US system 200 includes a workstation 205 for implementing and/or managing the processes described herein with regard to generating high-quality B-mode US images using harmonic signals, and accurate acoustic attenuation coefficient estimation using fundamental signals. In accordance with a representative embodiment, and as described herein, the US system may be an EPIQ ultrasound imaging system commercially available from Koninklijke Philips N.V. As will be described more fully below, the US system is adapted to function in HGEN mode providing both harmonic and fundamental components for use in imaging in the harmonic mode, and attenuation imaging and acoustic attenuation coefficient determination using the fundamental components of the ultrasound signal.

The workstation 205 includes one or more processors indicated by processing unit 220, one or more memories indicated by memory 230, a user interface 222, and a display 224. The processing unit 220 communicates with representative ultrasound imaging system 240 through an imaging interface (not shown). The ultrasound imaging system 240 includes an ultrasound controller 243 and an ultrasound probe 245 operable by an operator to obtain ultrasound images of various anatomies of a subject (patient) 250. The ultrasound probe 245 may be manipulated manually by the ultrasound user, automatically by a robot under control of a robot controller (not shown), or a combination of both.

The ultrasound probe 245 may include a 1D/2D matrix array of transducer elements, capable of scanning in two or three dimensions, for example, for emitting ultrasound waves into the body of the subject 250 and receiving echo signals in response. The transducer elements may include capacitive micromachined ultrasonic transducers (CMUTs) or piezoelectric transducers formed of materials such as lead zirconate titanate (PZT) or polyvinylidene difluoride (PVDF), for example, although other types of transducer material may be incorporated without departing from the scope of the present teachings. The transducer array may be coupled to a microbeamformer in the ultrasound probe 245, which controls transmission and reception of signals by the transducer elements.

The ultrasound probe 245 is connected to the ultrasound controller 243 via a probe cable 247. The ultrasound controller 243 is configured to control the ultrasound imaging process, and includes known elements for performing ultrasound imaging, such as a transmit/receive (T/R) switch configured to switch between transmission and reception modes, and a main beamformer configured to provide final beamforming. One of the functions performed by the ultrasound controller 243 is the direction in which beams are steered and focused. Generally, the transmitting of ultrasound signals and the receiving and processing of echo signals in response are known, and therefore additional detail in this regard is not included herein. In various embodiments, all or part of the functionality of the ultrasound controller 243 may be implemented by the processing unit 220.

The memory 230 stores instructions executable by the processing unit 220. When executed, the instructions cause the processing unit 220 to implement one or more processes of various representative embodiments. For example, and as described more fully below, the various steps carried out by the US system to separate fundamental and harmonic components from raw RF data, process these signals to provide high-quality B-mode images from the harmonic components of the raw RF data, accurate determine attenuation coefficients from the fundamental components of the raw RF data by calibration using a phantom image, the generation of an attenuation image from the fundamental components of the raw RF data, and the superposition of the high-quality B-mode image and attenuation image/acoustic attenuation coefficient are carried out by execution of computer executable instructions using a processor of a processing unit 220. As will be appreciated by one of ordinary skill in the art, these instructions are generated using known coding techniques on known platforms/machine languages.

The ultrasound images, including B-mode images and attenuation images comprising maps of acoustic attenuation coefficients of a particular tissue being evaluated, may be provided from the ultrasound imaging system 240 in real-time or near real-time during a scanning procedure, or may be retrieved from storage following the scanning procedure. For purposes of illustration, the memory 230 is shown to include software modules, each of which includes the instructions, executable by the processing unit 220, corresponding to an associated capability of the US system 200, as discussed below.

The processing unit 220 is representative of one or more processing devices, and may be implemented by a general-purpose computer, a central processing unit (CPU), a digital signal processor (DSP), a graphical processing unit, a computer processor, a microprocessor, a state machine, programmable logic device, field programmable gate arrays (FPGAs), application specific integrated circuits (ASICs), or combinations thereof, using any combination of hardware, software, firmware, hardwired logic circuits, or combinations thereof. Any processor or processing unit herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 230 may include main memory and/or static memory, where such memories may communicate with each other and the processing unit 220 via one or more buses. The memory 230 may be implemented by any number, type and combination of random access memory (RAM) and read-only memory (ROM), for example, and may store various types of information, such as software algorithms, AI machine learning models, and computer programs, all of which are executable by the processing unit 220. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 230 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 230 may store software instructions and/or computer readable code that enable performance of various functions. The memory 230 may be secure and/or encrypted, or unsecure and/or unencrypted.

The US system 200 may also include databases for storing information that may be used by the various software modules of the memory 230. Representative databases may include an image database 212 for storing images from current and previous examinations of subjects. The image database 212 may include image data from previously obtained ultrasound images of various subjects. Each of the image database 212 may be implemented by any number, type and combination of RAM and ROM, for example, as discussed above with regard to the memory 230. The databases comprise tangible storage mediums for storing data and executable software instructions and is non-transitory during the time data and software instructions are stored therein. The databases may be secure and/or encrypted, or unsecure and/or unencrypted. For purposes of illustration, the image database 212 is shown as separate storage media, although it is understood that all or some of the databases may be combined with one another and/or included in the memory 230, without departing from the scope of the present teachings.

The user interface 222 is configured to provide information and data output by the processing unit 220, the memory 230 and/or the ultrasound imaging system 240 to the user and/or for receiving information and data input by the user. That is, the user interface 222 enables the user to enter data and to control or manipulate aspects of the processes described herein, and also enables the processing unit 220 to indicate the effects of the user's input, which may include control or manipulation of the ultrasound probe 245 by the user. All or a portion of the user interface 222 may be implemented by a graphical user interface (GUI), such as GUI 228 viewable on the display 224. The user interface 222 may include one or more interface devices, such as a mouse, a keyboard, a trackball, a joystick, a microphone, a video camera, a touchpad, a touchscreen, voice or gesture recognition captured by a microphone or video camera, for example. Of note, the user interface allows the user (sonographer or other clinician) to select certain images for not only the particular examination, but also for archiving and later use in retraining or updating the model and user training and user performance evaluation.

The display 224 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 224 includes a screen 226 for viewing ultrasound images of the subject 250, along with various features described herein to communicate to the user the degree of image degradation, if any, as well as the GUI 228 to enable the user to interact with the displayed images and features. In an embodiment, the ultrasound imaging system 240 may include a separate dedicated display for acquiring the ultrasound images, where dedicated display is also represented by the display 224.

In the depicted illustrative embodiment, the memory 230 includes ultrasound imaging module 231, which is configured to receive and process ultrasound images acquired by the ultrasound probe 245 of the ultrasound imaging system 240 to provide corresponding ultrasound image data. The ultrasound images are obtained by the user during corresponding ultrasound examinations, e.g., of the subject 250. The ultrasound images may be received in real-time or near real-time from the ultrasound imaging system 240, e.g., during a contemporaneous imaging session of the subject 250. The ultrasound images are displayed on the display 224, enabling the user to visualize the anatomy of the subject 250 while performing sweeps using the ultrasound probe 245. Alternatively, or additionally, the ultrasound images may be previously acquired images, obtained during a previous imaging session of the subject 250 and retrieved from storage (e.g., image database 212). The ultrasound imaging module 231 may also store data associated with the ultrasound images, such as time and date of image acquisition, identification of the ultrasound imaging system 240, and identification of the operator who acquired the ultrasound images using the ultrasound imaging system 240.

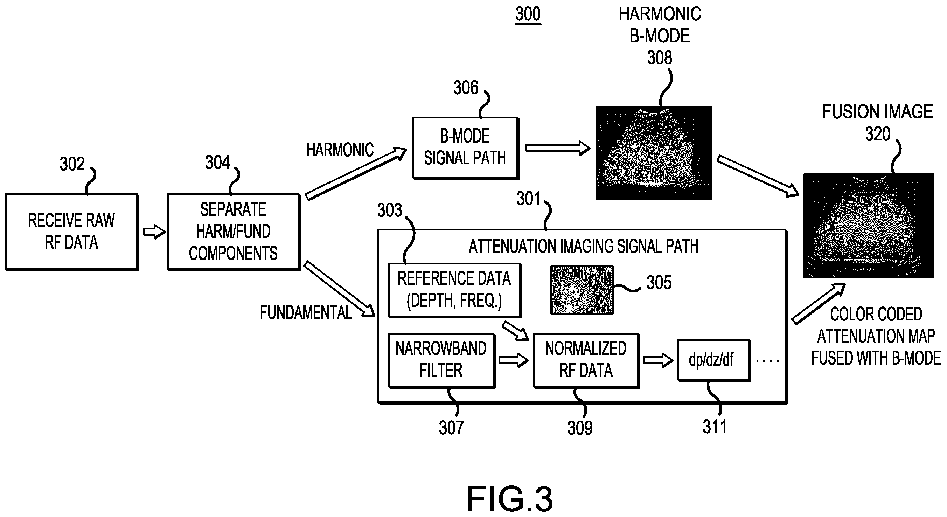

Fig. 3 is a flow-chart 300 showing the function of the US imaging system of Fig. 2 in accordance with a representative embodiment. Various aspects and details described in connection with Fig. 3 may be common to those described above in connection with Fig. 2 and may not be repeated to avoid obscuring the presently described representative embodiment. As alluded to above, the various functions described in connection with the flow-chart 300 are carried out by execution by the processing unit 220 of computer-executable instructions stored in memory 230.

Raw radio frequency (RF) data are received at 302 by the ultrasound controller 243 from the ultrasound probe 245. These RF data include fundamental components and harmonic components as noted above. At 304, the raw RF data are separated by a known technique to provide an attenuation imaging signal path 301 comprising the fundamental components and a harmonic signal path (B-mode path) 306 comprising harmonic signals. In accordance with a representative embodiment, a pulse inversion sequence is used to achieve harmonic mode imaging, with fundamental components being extracted by performing the opposite operation that is typically used for suppressing the fundamental components in harmonic mode imaging. Illustratively, this is done by subtracting (instead of summing) the receive (RX) signals from the two inverted transmit pulses. As a result, both fundamental and harmonic components (resulting from subtracting and summing RX data beamformed from a pair of inverted transmit (TX) pulses) are restored in the post- Transmit Beamforming Retrospective (XBR) data structure, which refers to compounding the received data from multiple transmits. Notably, the XBR data structure may be a data tap-point available for on-cart implementation. Similar approaches can be taken to extract fundamental and harmonic signal components if amplitude modulation techniques are used for tissue harmonic imaging, or to apply filters on the signals to separate the fundamental and harmonic components.

Referring first to the B-mode path 306, harmonic mode images are generated at 308 by known techniques for display for example at display 224. As noted above, the harmonic mode images provide improved image quality compared to images generated using fundamental mode imaging. As described more fully below, the images generated in the B-mode path 306 are later fused with an attenuation map generated in the attenuation imaging signal path 301, where the attenuation map includes a plurality of attenuation coefficients from the fundamental components gathered from the raw RF data received at 302.

Turning to the attenuation imaging signal path 301, the fundamental signals are used to generate the attenuation map from the broadband fundamental components by breaking the broadband signal into a plurality of narrowband signals and calculating a plurality of acoustic attenuation coefficients at the different frequencies, which are then combined to provide a robust attenuation map/image for fusing with the harmonic mode images generated at 308 for display at display 224, for example. Accordingly, the attenuation imaging signal path 301 provides attenuation imaging by measuring how the intensity of different frequency components changes with depth in the tissue. Notably, because the acquired signal is influenced by the characteristics of the US system 200, a calibration sequence is required to maintain the accuracy of the acoustic attenuation coefficient calculations and corresponding acoustic attenuation image.

At 303, reference data are used to provide a baseline measurement of how different frequency components behave in a medium with a known attenuation coefficient. These reference data are derived from the harmonic mode dataset acquired from a phantom image. To obtain this reference, narrowband filters are applied on the fundamental components to extract the intensity of different frequency bands.

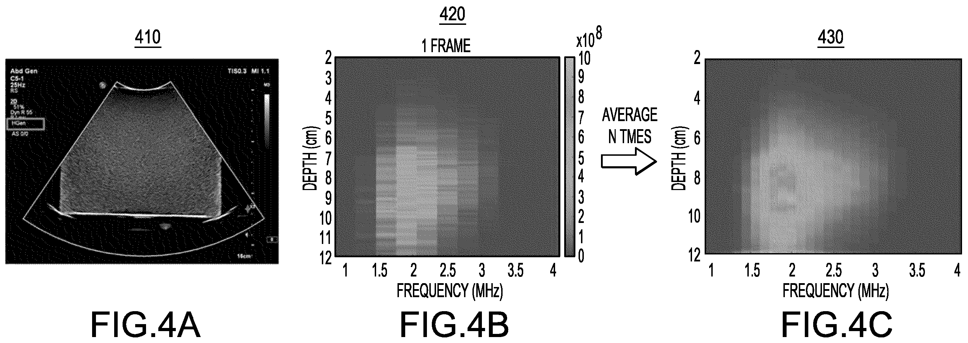

At 305, the reference data from the phantom image are provided as a 2D plot showing how intensity varies with depth and frequency. This calibration sequence improves the attenuation coefficient calculations to substantially ensure the remaining data are the actual attenuation due to the tissue, and not due to the imaging system. Specifically, to compensate for the energy variation as a function of depth induced by the system beam pattern and acoustic settings, reference phantoms with known acoustic attenuation coefficients are typically used in the calibration process. As will be appreciated, it is beneficial to have the same system acquisition settings between the calibration process and the application of attenuation imaging in real-time. This requires the calibration data to be acquired in the harmonic imaging mode, while the power spectrum is derived using the fundamental signals. As described more fully in connection with Figs. 4A-4C, the power spectrum of a uniform phantom with a known acoustic attenuation coefficient is used in the calibration sequence. Further details of a calibration method contemplated for use in accordance with various representative embodiments described herein may be found in commonly owned U.S. Patent 12,016,730 entitled "Ultrasound Imaging of Acoustic Attenuation Coefficient with Confidence Estimation" to Sheng-Wen Wang, et al. The entire disclosure of U.S. Patent 12,016,730 is specifically incorporated herein by reference.

After acquiring the fundamental components of the RF test data, at 307, the same narrowband filters as those used for the reference data are applied to the raw RF fundamental components received from 304. This ensures that the test data and reference data are treated identically. At 309, a normalization step is carried out. This normalization is done by dividing the test data by the reference data, thereby removing system dependencies.

At 311, the attenuation coefficient is obtained by taking the derivative of the normalized data with respect to depth and frequency (dP/dz/df). These data are then superimposed on the harmonic mode image 308 (B-mode image) to provide a fused image at 320.

At 320, the attenuation map generated in the attenuation imaging signal path 301, which is superimposed on the B-mode image from the harmonic signal path, is displayed. As will be appreciated, the superimposed images provide the comparatively accurate acoustic attenuation coefficient images with the comparatively high-quality B-mode images. As such, a high image quality for the B-mode harmonic mode image is realized, with the robust performance for the attenuation imaging right being maintained.

Accordingly, and as alluded to above, harmonic signals propagate differently in tissue compared to phantom while fundamental signals are fairly consistent. As such, use of a reference phantom imaged with fundamental frequencies are used to eliminate system dependencies. As such, by the present teachings, fundamental signals of the harmonic mode imaging system are used for attenuation imaging.

Fig. 4A shows a B-mode image of a uniform phantom, and Figs. 4B-4C show the power spectrum of a uniform phantom with a known acoustic attenuation coefficient. Various aspects and details described in connection with Figs. 4A-4C may be common to those described above in connection with Figs. 2 and 3 and may not be repeated to avoid obscuring the presently described representative embodiment. Turning to Fig. 4A, a B-mode image 410 of the phantom generated in harmonic imaging mode. Specifically, Fig. 4A illustrates the step of collecting data by imaging a uniform phantom in harmonic mode. The raw data from this mode comprises both fundamental and harmonic signals.

Fig. 4B shows a power spectrum versus frequency and depth of a single frame 420 of the phantom. Specifically, the RF data were acquired in harmonic imaging mode with the fundamental signal components being used to calculate the power spectrum as function of depth and frequency for calibration. As shown, a region of comparatively high attenuation is centered at approximately 20 MHz at a depth of approximately 9 cm from the ultrasound probe.

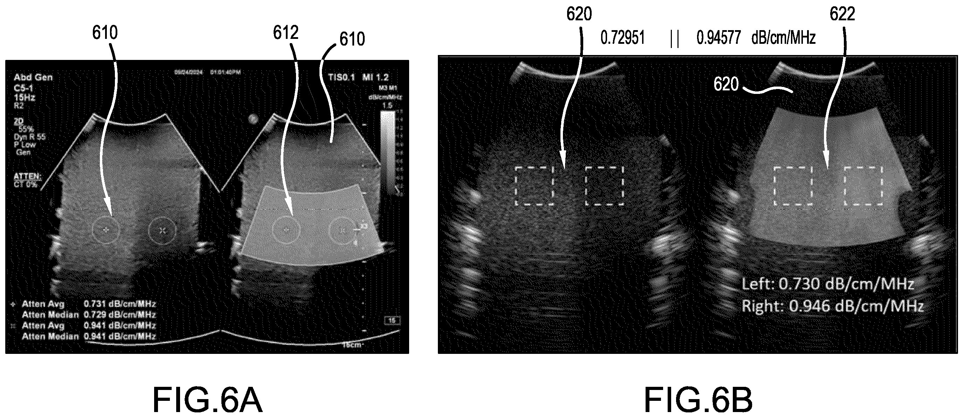

Fig. 4C shows an averaged power spectrum 430 over N acquisitions of different phantom image planes to take into account speckle variations. Again, the RF data were acquired in harmonic imaging mode, and the fundamental signal components were used to calculate the power spectrum as a function of depth and frequency for calibration. To remove speckle-induced variation, multiple acquisitions (N on the order of 50) of different image planes in the same phantom are required for power spectrum. As shown in connection with Fig. 6B below, the power spectrum of Fig. 4C is used to generate the attenuation image that is superimposed on the B-mode image, with both images being generated in harmonic mode imaging. During attenuation imaging, the power spectrum of the media or tissue is generated and normalized against this reference power spectrum, thereby substantially eliminating system dependency. The normalized power spectrum of the tissue is then used to derive the attenuation coefficients in the media.

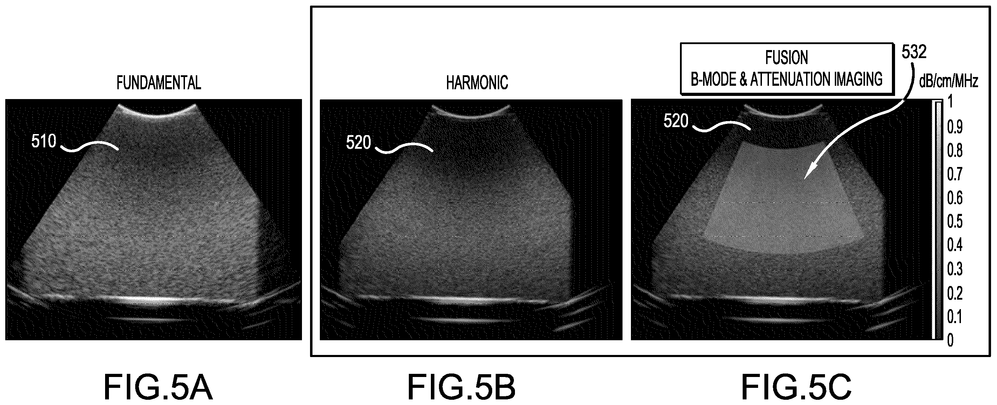

Figs. 5A-5C displays three output images from a US system and method of a representative embodiment. Fig. 5A shows B-mode image 510 from fundamental signals, which is of poor image quality compared to the B-mode from harmonic signals as shown in Fig. 5B and previously at image 410 in Fig. 4A. Moreover, Fig. 5C shows a fusion map image having an attenuation map 532 derived from the fundamental signals superimposed on the harmonic B-mode image 530 for quantitative measurement in accordance with various representative embodiments described above. It is evident from Fig. 5B that the B-mode image has higher spatial resolution (i.e., tighter speckle size) than the fundamental B-mode image 510 of Fig. 5A. Illustratively, in a deployed US system, the images of Figs. 5B-5C, will be displayed on the scanner monitor to the user.

Figs. 6A and 6B are US images showing equivalent attenuation coefficient estimation performance and displays using a known US imaging system, and an US imaging system in accordance with a representative embodiment. Various aspects and details described in connection with Figs. 6A-6B may be common to those described above in connection with Figs. 2-5C and may not be repeated to avoid obscuring the presently described representative embodiment.

Fig. 6A shows an equivalent acoustic attenuation coefficient estimation performance of a known commercial US image on a phantom. Specifically, a B-mode image 610 that is generated using fundamental mode imaging is shown on the left side of Fig. 6A. An attenuation image 612 is shown superimposed on the B-mode image on the right side of Fig. 6A. The phantom has two uniform segments with two different acoustic attenuation coefficients of 0.731 dB/cm/MHz and 0.941 dB/cm/MHz.

Fig. 6B shows an equivalent acoustic attenuation coefficient estimation performance of a US image on the same phantom used for Fig. 6A, but using harmonic mode imaging according to a representative embodiment. Specifically, a B-mode image 620 that is generated using harmonic components is shown on the left side of Fig. 6B. An attenuation image 622 is shown superimposed on the B-mode image on the right side of Fig. 6B. The phantom has two uniform segments with two different acoustic attenuation coefficients of 0.730 dB/cm/MHz and 0.946 dB/cm/MHz.

As will be appreciated from a review of Figs. 6A and 6B, the B-mode image 620 generated by harmonic frequencies of harmonic mode has better spatial resolution than the B-mode image 610 generated by fundamental mode imaging. However, unlike the comparatively poor attenuation images generated when harmonic components are used to generate the attenuation image, the attenuation image 622 generated using the fundamental frequencies of the harmonic mode imaging system of the present teachings is comparatively substantially the same as the attenuation image 612 generated solely using fundamental mode imaging. Accordingly, and among other benefits, a higher degree of resolution of the B-mode image 620 with substantially no decrease in the accuracy the attenuation image 622 is realized in accordance with various representative embodiments.

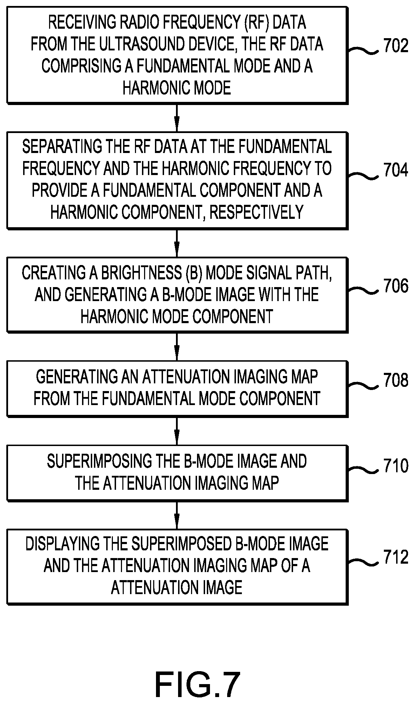

Fig. 7 is a flowchart of a method of performing a US exam in accordance with a representative embodiment. Various aspects and details described in connection with Fig. 7 may be common to those described above in connection with Figs. 2-6B and may not be repeated to avoid obscuring the presently described representative embodiment. As will be appreciated, the method described presently may be instantiated in software and comprise computer executable instructions to carry out the illustrative method.

At 702, the method includes receiving radio frequency (RF) data from the ultrasound probe, the RF data comprising a fundamental mode and a harmonic mode.

At 704, the method further includes separating the RF data acquired from the harmonic mode to provide a fundamental component and a harmonic component, respectively.

At 706, the method further includes creating a brightness (B) mode signal path, and generating a B-mode image with the harmonic component.

At 708, the method further includes generating an attenuation imaging map from the fundamental component.

At 710, the method further includes superimposing the B-mode image and the attenuation imaging map.

At 712, the method further includes displaying the superimposed B-mode image and the attenuation imaging map of a tissue attenuation image.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. An ultrasound system (200) for tissue attenuation imaging, the ultrasound system comprising:
an ultrasound probe (245);
a processor (220); and
a tangible, non-transitory computer readable medium (230) that stores instructions, which when executed by the processor, cause the processor to:
receive radio frequency, RF, data (302) from the ultrasound probe (245), the RF data comprising a fundamental mode and a harmonic mode;
separate the RF data comprising the fundamental mode and the harmonic mode to provide a fundamental component and a harmonic component, respectively;
create a brightness, B, mode signal path (306), and generate a B-mode image (308) based on the harmonic component;
create an attenuation signal path (301), and generate an attenuation imaging map based on the fundamental component;
superimpose the B-mode image and the attenuation imaging map; and
display the superimposed B-mode image and the attenuation imaging map of a tissue attenuation image (320).

2. The ultrasound system of claim 1, wherein before the superimposing, the instructions further cause the processor to calibrate the attenuation imaging map (532, 622) using a set of reference data acquired from a phantom having a known acoustic attenuation coefficient.

3. The ultrasound system of claim 2, wherein the calibration comprises acquiring calibration data in a harmonic imaging mode.

4. The ultrasound system of claim 3, wherein the instructions further cause the processor to separate the fundamental component from a broadband signal and average the fundamental component over multiple calibration data (430).

5. The ultrasound system of claim 3 or 4, wherein intensities of the fundamental component for different frequency bands are used to eliminate system dependencies.

6. The ultrasound system of any of claims 2 to 5, wherein the phantom is a uniform phantom.

7. The ultrasound system of any of claims 1 to 6, wherein the instructions further cause the processor to generate the harmonic component by providing a pair of ultrasound pulses that are inverted 180° out of phase, and sum the ultrasound pulses to substantially cancel the fundamental component.

8. The ultrasound system of claim 7, wherein the instructions further cause the processor to subtract the pair of ultrasound pulses to obtain the fundamental component and cancel the harmonic component.

9. The ultrasound system of claim 7 or 8, wherein the pair of ultrasound pulses are provided in sequence to one another.

10. A tangible, non-transitory computer-readable medium that stores instructions (230), which when executed by a processor (220), causes the processor to:
receive radio frequency (RF) data (302) from the ultrasound probe (245), the RF data comprising a fundamental mode and a harmonic mode;
separate the RF data comprising the fundamental mode and the harmonic mode to provide a fundamental component and a harmonic component, respectively;
create a brightness (B) mode signal path (306), and generate a B-mode image (308) based on the harmonic component;
create an attenuation signal path (301), and generate an attenuation imaging map based on the fundamental component;
superimpose the B-mode image and the attenuation imaging map; and
display the superimposed B-mode image and the attenuation imaging map of a tissue attenuation image (320).

11. The tangible, non-transitory computer readable medium of claim 10, wherein before the superimposing, the instructions further cause the processor to calibrate the attenuation imaging map (532, 622) using a set of reference data acquired from a phantom having a known acoustic attenuation coefficient.

12. The tangible, non-transitory computer readable medium of claim 11, wherein the calibration comprises acquiring calibration data in a harmonic imaging mode.

13. The tangible, non-transitory computer readable medium of claim 12, wherein the instructions further cause the processor to separate the fundamental component from a broadband signal and average the fundamental component over multiple calibration data (430).

14. The tangible, non-transitory computer readable medium of claim 12 or 13, wherein intensities of the fundamental component for different frequency bands are used to eliminate system dependencies.

15. A computer-implemented method for tissue attenuation imaging, comprising:
receiving radio frequency, RF, data (302) from an ultrasound probe (245) of an ultrasound system (200), the RF data comprising a fundamental mode and a harmonic mode;
separating the RF data comprising the fundamental mode and the harmonic mode to provide a fundamental component and a harmonic component, respectively;
creating a brightness, B,-mode signal path (306), and generating a B-mode image (308) based on the harmonic component;
creating an attenuation signal path (301), and generating an attenuation imaging map based on the fundamental component;
superimposing the B-mode image and the attenuation imaging map; and
displaying the superimposed B-mode image and the attenuation imaging map of a tissue attenuation image (320).
